# EUROPEAN PATENT SPECIFICATION

(11) **EP 2 959 862 B1**
(45) Date of publication and mention of the grant of the patent: **29.06.2022**
(21) Application number: 15173417.5
(22) Date of filing: 23.06.2015
(51) Int. Cl.: A61C 8/02, A61F 2/00, A61F 2/28

(54) **PERFORATED MEMBRANE FOR GUIDED BONE AND TISSUE REGENERATION**
PERFORIERTE MEMBRAN ZUR GEFÜHRTEN KNOCHEN- UND GEWEBEREGENERATION
MEMBRANE PERFORÉE POUR LA RÉGÉNÉRATION OSSEUSE ET TISSULAIRE GUIDÉE

(30) Priority: 24.06.2014 US 201414313685
(43) Date of publication of application: 30.12.2015
(73) Proprietor: Osteogenics Biomedical Inc., Lubbock, TX 79424 (US)
(72) Inventor: BARTEE, Barry K., Lubbock, TX Texas 79423 (US); BARTEE, Chad M., Lubbock, TX Texas 79423 (US); URBAN, István, Culver City, CA California 90230 (US)
(74) Representative: Lantos, Mihaly

(56) References cited:
- DE-A1-102012 024 206
- US-A1- 2008 044 449
- US-A1- 2010 217 392
- US-B1- 6 409 764

## Description

### FIELD

This disclosure relates to a membrane configured to guide bone and tissue regeneration for a bone defect.

### BACKGROUND

It is widely known that the occurrence of tooth loss in human dentition (which may happen as the result of dental diseases, advanced age, genetic inclination, accidents, etc.) is the cause of a number of functional and aesthetic problems. For example, if chewing is unsatisfactory, the entire digestive system may be affected, and the unsatisfactory chewing may cause gastrointestinal dysfunction and complaints.-From an aesthetic point of view, properly cared for teeth have considerable significance.

Previously, missing teeth were replaced with a partial denture or a permanently attached dental bridge, supported in part by the remaining natural teeth. Bridges, however, are multi-piece inflexible systems, the shape and color of which do not always conform to expectations, and their participation in the chewing process is also frequently imperfect. Contemporary tooth replacement includes the extraction of damaged or hopeless teeth and implantation titanium implants supported by the patients own natural bone. If there is inadequate bone for implant support, this bone may be reconstructed using autogenous bone grafts. This, however, is an expensive procedure, which may require hospitalization and in certain cases possible complications. Other options for bone reconstruction include guided tissue regeneration (GTR) and bone regeneration (GBR). Both of these techniques involve the regeneration of bone deficiencies affecting natural teeth by means of barrier membranes. GTR implies the regeneration of the bone and attachment apparatus (ligaments, cementum) of natural teeth, whereas GBR includes the implantation of a membrane into the location where the formation of bone is intended. For either technique, bone and/or bone replacement material is typically used under the membrane. Currently, the membranes used for GTR/GBR do not include macro-perforations in the membrane, but rather are engineered to be cell-occlusive while simultaneously being able to allow the passage of small molecules. It is widely accepted that the membrane functions as a barrier to protect the healing environment form soft tissue ingrowth and resorptive stimuli.

DE 10 2012024206 A1 describes a membrane for bone formation which comprises at least two connected layers of which a first layer is a contact surface and the other layer is at the opposite side of the membrane. The two layers are made of different porous materials and the size of the pores are chosen so that when the layers contact each other certain pores provide channels (openings) interconnection the two faces of the membrane.

US 6,409,764 B1 describes methods and articles for regenerating bone or periodontal tissues which comprises a tissue penetrable device that has a plurality of holes therethrough that permit cells and vascular structures to grow through the device, which can be made of either a non-degradable material including expanded PTFE or of a degradable material like collagen. The disclosed articles are used in combination with members of the Transforming Growth Factor-Beta Superfamily of proteins (TGF-Beta proteins),

From this publication it is known that perforations through the membrane can have beneficial effect only if used together with TGF-Beta proteins.

US 2010/217392 A1 relates to a membrane configured to guide bone and tissue regeneration and describes a pair of porous membranes made of respective flexible sheet materials, of which the outer layer is made of dense unsintered PTFE that has a smooth outer surface that provides tissue excluding (TE) properties to the membrane and facilitates removal of the membrane after the bone regeneration has terminated. The pores of this dense PTFE layer allow only the passage of ions and molecules and retains the passage of cells (including bacteria) and ingrowth of soft tissues. This publication is an example for the previously mentioned type where the membrane functions as a barrier.

It is an object of the present invention to provide a membrane which is a modification of traditional GTR/GBR membranes designed to increase the potential for communication between cells with bone forming potential and the periosteum, while simultaneously provide stability and protection of the three-dimensional envelope of space so that bone may form guided by the final contour of the membrane and to prevent connective tissue fibers from growing into the bone tissue.

### SUMMARY

The invention lies in a membrane configured to guide bone and tissue regeneration for a bone defect. The membrane is as defined by the appended independent claim 1.

Preferred embodiments are structured as defined in the attached dependent claims.

### BRIEF DESCRIPTION OF THE DRAWINGS

FIG. 1 illustrates a membrane configured to guide bone and tissue regeneration for a bone defect.
(12) FIG. 2 illustrates a membrane without a reinforcement binder.
(13) FIG. 3 illustrates first layer of the membrane.
(14) FIG. 4 illustrates a second layer of the membrane.
(15) FIG. 5 illustrates a texture pattern formed in the second layer of the membrane.
(16) FIG. 6 illustrates an example implementation of a reinforcement binder.
(17) FIG. 7 illustrates an example implementation of the reinforcement binder.
*(18)* FIG. 8 is a lateral cross-sectional view of an adult human maxilla after a tooth extraction showing alveolar bone.
(19) FIG. 9 shows a tooth socket packed with bone and covered with a membrane.
*(20)* FIG. 10 shows a typical tooth extraction site after healing, but prior to removal of a membrane.
(21) FIG. 11 illustrates a method for guiding bone and tissue regeneration for a bone defect with a membrane.
(22) FIG. 12 illustrates an example of the membrane wherein the second layer includes perforations but the first layer does not.
(23) FIG. 13 illustrates treatment of a discontinuity defect with the membrane.
(24) FIG. 14 illustrates treatment of a vertical defect with the membrane.
(25) FIG. 15 illustrates treatment of a horizontal defect with the membrane.

### DETAILED DESCRIPTION

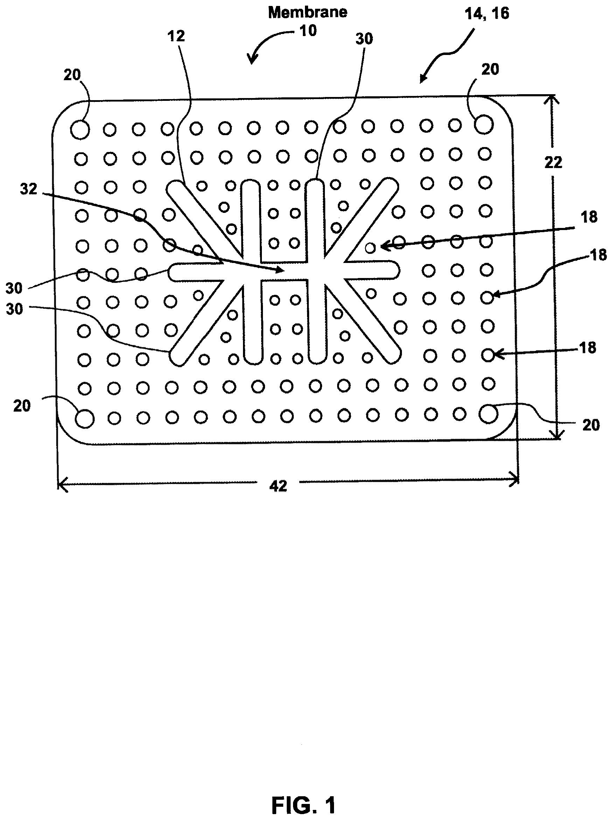

FIG. 1 illustrates membrane 10. Membrane 10 may be configured to guide bone and tissue regeneration for a bone defect. Membrane 10 may be suitable for guided bone regeneration (GBR), guided tissue regeneration (GTR), and/or other therapies. Membrane 10 may form a barrier membrane used for guiding bone and tissue regeneration for dental and/or other purposes. Membrane 10 may be used in oral surgery, maxillofacial surgery, craniofacial surgery, to treat periodontal diseases, for dental implants, to treat orbital floor bone defects, and/or for other applications. Membrane 10 may be configured to maintain space for bone regeneration, to prevent connective tissue fibers from growing into the bone tissue, to immobilize bone, to exclude stimuli, which may hinder bone generation, and/or for other purposes. Membrane 10 may be a barrier membrane that is applicable in alveolar ridge defect replacements and/or for other defects that membrane 10 is sized to cover. Membrane 10 may facilitate reducing and/or eliminating injuries, and/or facilitate a more efficient bone and tissue regeneration process compared to previously known techniques. Membrane 10 may include perforations 18 located at various distances from each other throughout membrane 10.

Perforations 18 are provided in the membrane 10 based on the surprising discovery that if a barrier membrane does not constitute a continuous surface at the location of the desired bone and tissue regeneration (e.g. if the membrane includes perforations that are larger than a pore size in a surface of the membrane) the membrane still induces a positive effect on bone regeneration, especially when combined with the use of various biological growth factors. Membranes with perforations have been shown to better facilitate bone regeneration than membranes without perforations. The perforated design also makes the membrane easier to handle, fixate, and/or shape during application because the surgeon may more readily visualize, for example, pilot holes made specifically for the purpose of securing membrane fixation screws, pins or tacks.

A perforated membrane may facilitate communication between a patient's periosteum and growth factors used with GBR. The growth factors may include RhPDGF (Platelet Derived Growth Factor), RhBMP (Bone Morphogenetic Protein), and/or other growth factors. The perforations may also facilitate communication between the periosteum and undifferentiated stem cells, especially in the presence of simulative growth factors. Application of a non-perforated collagen (for example) membrane may reduce the regenerative potential of PDGF. If a surgeon (and/or other users) utilizes ground autogenous bone for bone generation, which makes the application of a membrane necessary, the "permeability" of a perforated membrane may make the development of a connection with the periosteal membrane possible using the PDGF technique.

Similarly, membrane 10 may be used with RhBMP. Using membrane 10 with RhBMP may allow a surgeon (and/or other users) to avoid using a titanium net/mesh (used for its permeability relative to a non-perforated membrane), for example. A titanium net/mesh may cause complications because it is sharp and difficult to handle. Such complications may include, for example, damaging the gum of a patient or difficult removal (taking as long as thirty minutes). Conversely, membrane 10 may be removable in a matter of a few minutes, which decreases the duration of surgery and the possible occurrences of complications.

Continuing with the above non-limiting example comparison to the titanium net/mesh, the present invention differs from titanium mesh in several respects. These include mechanical compliance, placement, removal, customization, and/or other differences. (1) Mechanical Compliance: perforated PTFE is more flexible, and therefore has improved compliance with soft tissue compared to titanium mesh. Mechanical compliance is important because biomaterials that are compliant with soft tissue have a much less reduced risk of soft tissue dehiscence (opening) and/or wound healing complications. (2) Placement: because of its flexibility and softness, membrane 10 easier to adapt, place, and/or fixate compared to titanium mesh. (3) Removal: regenerated bone tends to grow through and/or over the titanium mesh struts, making it exceedingly difficult to remove. During removal of titanium mesh, damage to the immature regenerated bone may occur, resulting in less volume of regenerated tissue than desired. In contrast, with membrane 10 being flexible, it is much easier to remove and in fact is able to stretch and therefore presents less risk of damaging the newly formed bone tissue as the device is removed. (4) Customization: relating to method, in a 'custom fit' application, the present invention is much easier to trim and cut, and/or the surgeon may easily punch holes directly at the time of surgery with a simple hand punch, enabling the creation of a truly custom surgical device for individual defects. The surgeon may place the holes in exactly the location desired, and they may make more holes, or fewer holes depending on the clinical indication. For example, it may be advantageous in a case where membrane exposure was desired, to leave the exposed portion non-perforated and to create perforations in the areas where communication between the periosteum and graft bed was desired. This maneuver would be exceedingly difficult to accomplish with a sheet of titanium.

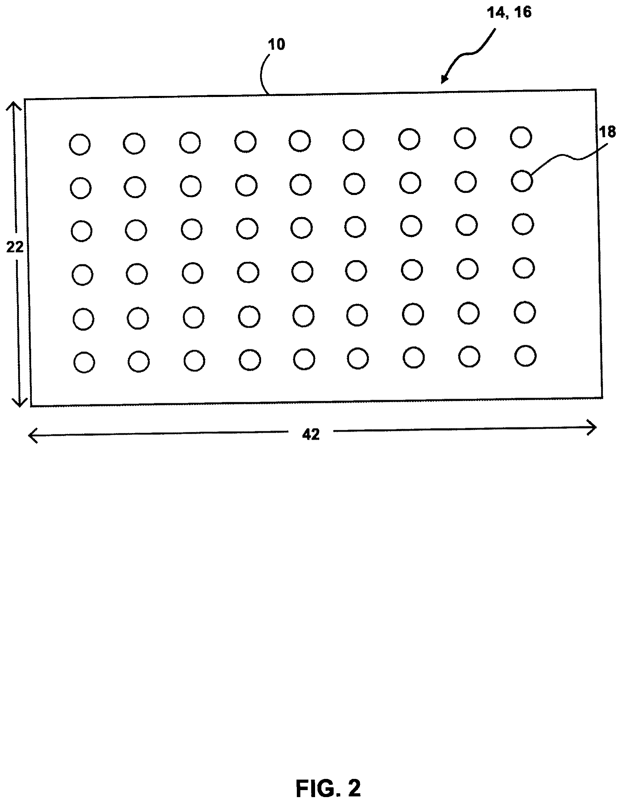

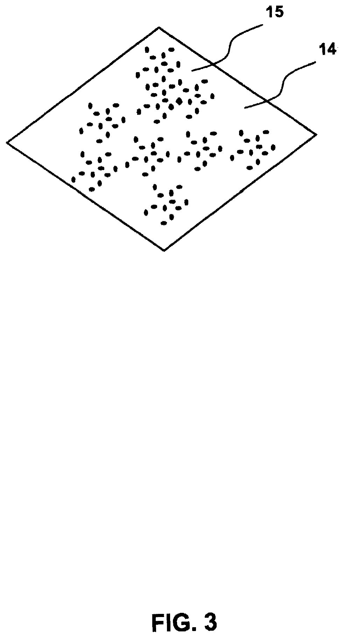

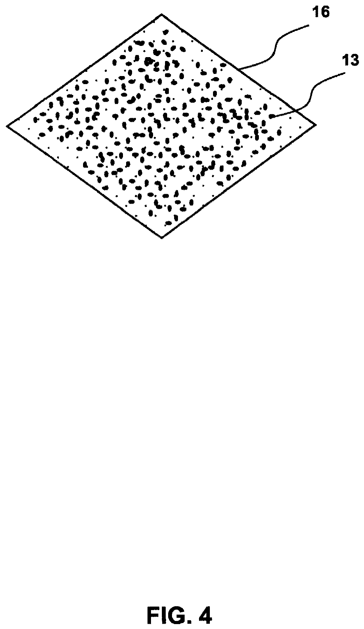

The membrane 10 includes a first layer 14 (FIG. 3), a second layer 16 (FIG. 4), one or more perforations 18, and may include a reinforcement binder 12, and/or other components. The first layer consists of a thin layer of expanded PTFE. Said membrane may or may not contain a titanium framework between the layers. In some implementations, membrane 10 may not include reinforcement binder 12. For example, FIG. 2 illustrates membrane 10 without reinforcement binder 12. First layer 14, second layer 16, reinforcement binder 12, and/or other components of membrane 10 may be similar to and/or the same as similar components described in United States Patent Number 8,556,990 granted on October 15, 2013, and entitled, "Reinforced PTFE Medical Barriers,".

As shown in FIG. 2, membrane 10 may be generally rectangular and have a length 42 and a width 22. In some implementations, length 42 may be less than about 50mm. Length 42 may be between about 30mm and about 50mm. Length 42 may be about 40mm. In some implementations, width 22 may be less than about 40mm. Width 22 may be between about 20mm and about 40mm. Width may be about 30mm. In some implementations, membrane 10 may have a thickness from about 0.125 mm to about 0.25 mm. The generally rectangular shape and approximate dimensions of membrane 10 shown in FIG. 2 are not intended to be limiting. Membrane 10 may take any shape and have any dimensions that allow it to function as described in the present disclosure.

The membrane 10 is formed from a first layer of expanded PTFE and a second layer of unsintered, substantially unexpanded PTFE. The term sintered is a term well known in the art and is used herein consistent with that understanding. The term unsintered is used herein to describe PTFE polymer that has not been subjected to the sintering process. Unsintered PTFE may be substantially unexpanded and typically contains no substantially defined internodal distance, which may substantially reduce its porosity relative to expanded PTFE. The limited porosity of the unsintered, substantially unexpanded PTFE may substantially reduce tissue adhesion to the unexpanded PTFE and/or migration of tissue into the unexpanded PTFE. However, the limited porosity may allow for the passage of ions and other small molecules necessary for cellular nourishment and waste transport. In some implementations, a density of one or more layers 14, 16 (FIG. 3 and FIG. 4) of membrane 10 may be about 1.2 gm/cc to about 2.3 gm/cc. In some implementations, the density of one or more layers 14, 16 of membrane 10 may be about 1.45 gm/cc to about 1.55 gm/cc.

FIG. 3 illustrates first layer 14 and FIG. 4 illustrates second layer 16. First layer 14 may be configured to contact bone. (This is not intended to be limiting. In some situations a user may place the first layer of the membrane in contact with soft and/or other non-bone tissue. For example, surgeons may choose to place the expanded PTFE layer or the dense PTFE layer towards bone, or soft tissue). First layer 14 includes pores 15 configured to promote ingrowth of bone regenerating cells into first layer 14. Second layer 16 may be fixedly coupled to first layer 14 and/or be coupled to first layer 14 in other ways. Second layer 16 is configured to substantially prevent fibrous connective tissue from growing into the bone defect. Second layer 16 comprises a dense structure that prevents tissue ingrowth. Second layer 16 is relatively denser than first layer 14, for example. First layer 14 and second layer 16 may be separate layers of membrane 10 (as described above), and/or first layer 14 and second layer 16 may be two surfaces on opposite sides of membrane 10 (e.g., opposite sides of a single layer).

The first layer 14 includes expanded PTFE (e-PTFE). The second layer 16 is and/or includes unsintered high density PTFE (d-PTFE) having a density of about 1.2 gm/cc to about 2.3 gm/cc. In some implementations, the density of d-PTFE may be in a range from about 1.45 grams/cc to about 1.55 grams/cc. The d-PTFE material is unsintered and unexpanded with a nominal pore channel 13 size of less than about 5 micrometers. In some implementations, the unsintered, unexpanded d-PTFE may have a nominal pore channel 13 size of less than about 2 micrometers. In some implementations, the unsintered, unexpanded d-PTFE may have a nominal pore channel 13 size of less than about 0.5 micrometers. In some implementations, the unsintered, unexpanded d-PTFE may have a nominal pore channel 13 size of less than about 0.2 micrometers. This small pore channel size may allow a composite multi-layer material employing d-PTFE to exhibit superior functional characteristics, resulting clinically in reduced host response (inflammation), soft tissue in-growth, and resultant adhesions. (These pore channel 13 sizes may be smaller than pore 15 sizes in first layer 14 made from e-PTFE that promotes bone ingrowth.)

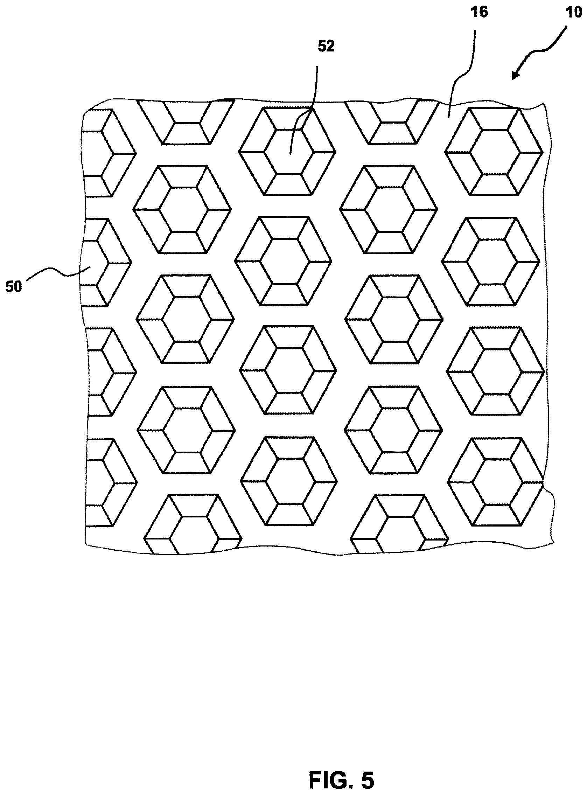

FIG. 5 illustrates a texture pattern 50 that may be formed in second layer 16 of membrane 10. The texture pattern may be formed by a plurality of indentations 52 formed in second layer 16 of membrane 10. Indentations 52 may have any shape that allows membrane 10 to function as described herein. The example shown in FIG. 5 is hexagonal in shape, although other shapes are contemplated and fall within the scope of this disclosure. The indentations may have a depth less than the thickness of second layer 16. In some implementations, indentations 52 may be up to about 0.15 mm deep and up to about 0.5mm wide, for example. Indentations 52 may be dimensioned based on the intended use for membrane 10 and/or other factors. The distribution of indentations 52 may be substantially uniform over second layer 16, may vary systematically across second layer 16, may be randomly distributed across second layer 16, and/or have other distributions. For example, up to about 150 indentations may be provided per square centimeter over second layer 16. As another example, up to about 250 indentations may be provided per square centimeter over second layer 16.

Texture pattern 50 of second layer 16 may be made by forming a thin sheet of PTFE and then embossing the sheet with indentations. PTFE resin may be mixed with a lubricant (e.g., mineral spirits) to form a paste. The paste may be calendered between rollers to form a thin flat sheet of the desired thickness (e.g., in the range of about 0.125 mm to about 0.25 mm.) The calendering may be performed to reduce the thickness of the sheet and to impart substantially uniform strength in all directions to the sheet. The lubricant may be removed by drying the sheet at a temperature somewhat above the boiling point of the mineral spirit lubricant, but well below the sintering temperature of PTFE. After the sheet has been dried, the sheet may be embossed to form the indentations in one of its surfaces. In some implementations, the embossing step may be performed by placing a sheet of patterned polymer mesh on top of the sheet of PTFE. The patterned polymer mesh may be harder and have more compressive strength than the PTFE material. In some implementations, the polymer mesh may be a suitable commercially available fine pore-size mesh material. The polymer mesh and the PTFE sheet may be passed together between a pair of rollers, which emboss the pattern of the polymer mesh into one surface of the PTFE sheet. After embossing, the polymer sheet may be discarded. One of many possible advantages of the textured surface is to increase the surface area available for cell attachment.

Returning to FIG. 1, perforations 18 comprise co-axial through-holes having common dimensions through first layer 14 and second layer 16. Perforations 18 may be configured to enhance ossification. The one or more perforations 18 are perforations with a substantially circular cross section having a diameter of about 0.1mm or larger. In some implementations, one or more perforations 18 may be and/or include perforations with a substantially circular cross section having a diameter of about 0.5mm to about 1.0mm. In some implementations, one or more perforations 18 may be and/or include perforations with a substantially circular cross section having a diameter of up to about 3.0mm. In some implementations, the shape and/or size of perforations 18 may vary across membrane 10. For example, perforations 18 may be smaller near reinforcement binder 12 and larger near the edges of membrane 10.

In some implementations, membrane 10 may be configured such that perforations 18 may be formed at manufacture, formed by a user (e.g., a surgeon, a doctor, a nurse, and/or other clinicians), and/or formed at other times. In some implementations, perforations 18 may be formed during an individual procedure according to the needs of the patient and/or the bone defect. For example, a user may use a sharp tool to perforate membrane 10, a tool associated with membrane 10, and or other devices to make perforations 18. In such implementations, the number and/or the spacing of the perforations may be determined by the user. In such implementations, the shape of perforations 18 may depend on the tool used to make the perforations.

One or more secondary perforations 20 are formed in membrane 10. Secondary perforations 20 are configured to receive fasteners configured to hold membrane 10 in place at the bone defect. Secondary perforations 20 may have a different size, shape, and/or density relative to perforations 18. The fasteners may be pins (e.g., Titanium Master Pins manufactured by the Meisinger corporation), titanium tacks (e.g., manufactured by Salvin), screws (e.g., manufactured by Pro-Fix, Osteogenics, etc.), and/or other fastening devices.

Reinforcement binder 12 may comprise multiple elongated members 30 extending from a junction 32. In some implementations reinforcement binder 12 may be formed between first layer 14 and second layer 16 of membrane 10. In some implementations, reinforcement binder 12 may be formed from titanium, stainless steel, platinum, ceramics, composites, carbon fiber materials, customized micro and/or nano material based materials, coated (e.g., with a non-toxic coating) materials, and/or other materials. Reinforcement binder 12 may be bendable and may include elongate members 30 such that reinforcement binder 12 may be formed in a desired shape (e.g. at manufacture), and/or may be bent, deformed, and/or reformed by a user to obtain the desired shape prior to placement about the bone defect such that the formed shape is maintained upon placement. For example, one or more portions and/or all of reinforcement binder 12 may be bent, twisted, and/or stretched as necessary to obtain the desired shape. In some implementations, reinforcement binder 12 may be malleable and/or flexible because it is relatively thin. For example, a thin piece of titanium may be easily bent by a user.

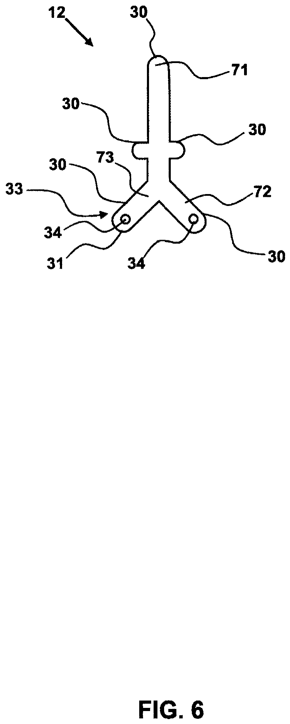

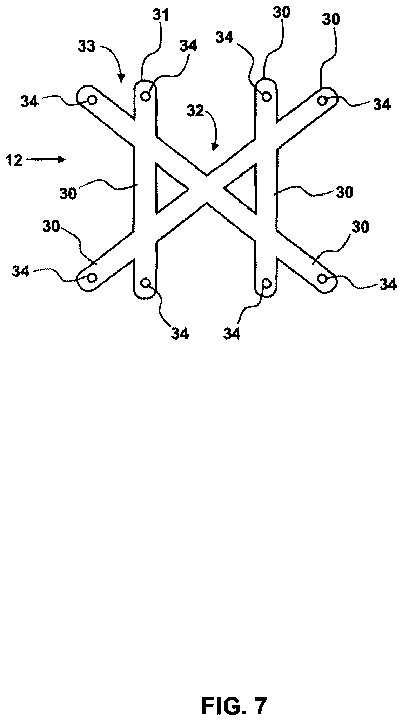

As shown in FIG. 6 and FIG. 7, elongated members 30 may include a first elongated member 31, for example, having a free end 33 that extends away from junction 32 with a predrilled hole 34 formed therein. In some implementations, one or more elongated members 30 may include predrilled holes 34. The pre-drilled holes may be suitable for securing a fastener such as a surgical pin or screw to the bone defect site. Reinforcement binder 12 may be placed over a bone cavity, such as an alveolar cavity, for example. FIG. 6 illustrates an example of reinforcement binder 12 which has an elongate member 71, for placement over a cavity toward a lingual side of the cavity. FIG. 6 illustrates two elongate members 72 and 73 in a "Y- shaped configuration" at an opposite end of reinforcement binder 12, both having holes 34 for securing a fastener. The fastener may be a surgical screw, for example, configured to fasten membrane 10 to an area of bone, typically at a surgical site, for example on the buccal side of the jaw, or upper alveolar arch, in the repair of alveolar defects and/or maxillofacial defects, for example.

Layers 14 and 16 (FIG. 3 and 4) may be coupled with reinforcement binder 12. Coupling may include fixing, attaching, and/or otherwise joining the layers and reinforcement binder 12 together. Layers 14, 16 and reinforcement binder 12 may be coupled using any suitable means, including use of an adhesive layer for attachment and/or bonding the layers 14, 16 and reinforcement binder 12. Layers 14, 16 may partially cover reinforcement binder 12. Layers 14, 16 may substantially envelope reinforcement binder 12.

Dimensions of reinforcement binder 12 may be selected based on the application (e.g., based on the bone defect to be treated). Similarly, the physical and mechanical properties of reinforcement binder 12 may be selected according to application. Titanium is used as the primary example herein. Surgical grade titanium may be used to provide malleability, strength, and low weight. It should be appreciated that titanium possesses strength and weight characteristics that, together with the biologically inert nature of the metal, offers advantages in many applications. It is contemplated that some applications may dictate that other dimensions, ratios of dimensions, and/or materials may be employed. For example, repair of bone material in a pelvis and/or a hip may require the use of steel and/or other materials.

The structural configuration of reinforcement binder 12 may be selected to facilitate ease of placement and/or use in reconstructive repair of bone defects of various sizes, related soft tissue repair, and/or skeletal surgery, for example. The structural configuration of reinforcement binder 12 may be selected to provide one or more appendages and/or elongate members suitable for placement about bone and/or surrounding tissue. The overall shape of reinforcement binder 12 may be selected to achieve a desired strength, load distribution, membrane support, placement of fasteners, comfort, ease of insertion and/or removal, and/or achieve other effects.

Membrane 10 and reinforcement binder 12 may be implemented at the bone defect. Implementing membrane 10 and reinforcement binder 12 at the bone defect may comprise placing membrane 10 and reinforcement binder 12 over the bone defect, receiving a fastener with the predrilled hole that passes through at least one of first layer 14 or second layer 16 of membrane 10, coupling membrane 10 and reinforcement binder 12 with surrounding bone, holding membrane 10 in place at the bone defect via the fastener, and/or other operations. In some implementations, reinforcement binder 12 may receive a shape imparted to reinforcement binder 12 via bending by a user (e.g., a surgeon). Membrane 10 may be placed over and/or about a bone defect (e.g., a bone cavity) and/or a target surgical site with the unsintered, textured d-PTFE (second layer 16) facing soft tissue (e.g., gingival tissue) and the expanded e-PTFE (first layer 14) facing and/or adjacent to the bone and/or skeletal cavity. This is not intended to be limiting. In some implementations, if a surgeon (for example) desires, and/or if the clinical situation dictates, the aforementioned orientation may be reversed such that second layer 16 faces bone and first layer 14 faces soft tissue.

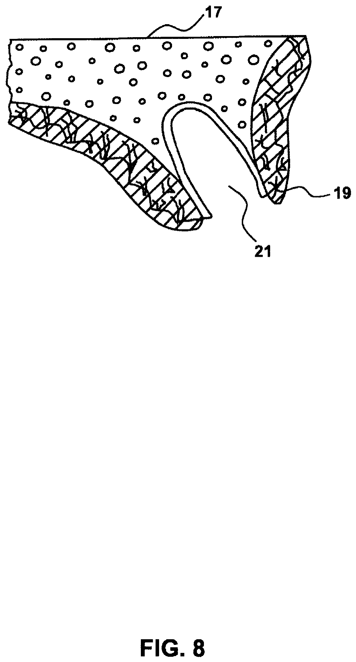

For example, FIG. 8 - 10 show examples of implementing membrane 10 at a bone defect. FIG. 8 is a lateral cross-sectional view of an adult human maxilla after a tooth extraction showing alveolar bone 17. Soft tissue gingiva 19 covers bone 17. A tooth socket 21 provides an example of a bone defect. Normal healing of a defect may include migration of cells such as fibroblasts and gingival epithelial cells, for example. As the cells proliferate into defect 21, they may inhibit bone cell regeneration, which may result in overall loss of bone mass. In the case of extractions, the loss of bone mass may result in a loss of the alveolar ridge profile.

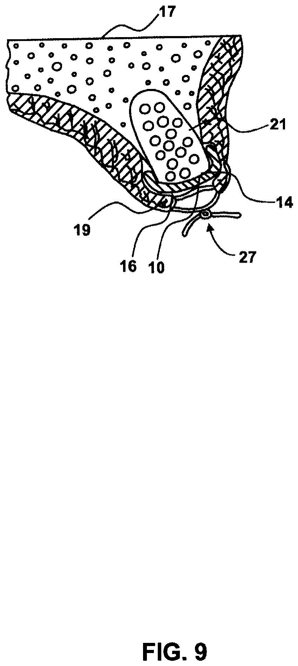

FIG. 9 shows socket 21 packed with bone and covered with membrane 10. Socket 21 may be packed with granular particles of allograft, xenograft and/or bioresorbable hydroxyapatite, for example, as a precursor to bone, and/or other materials. Other materials and/or articles, such as endosseous type dental implants, may be placed into socket 21. The packed socket 21 may be covered with membrane 10. First layer 14 may be placed over and/or facing socket 21 and/or bone 17. Second layer 16 may face and/or contact tissue 19 growing over and/or around membrane 10. After membrane 10 is placed over socket 21 and bone 17, membrane 10 (e.g., reinforcement binder 12) may be secured in place via fasteners (not shown). Gingival flaps 19 may be sutured 27 over membrane 10. Membrane 10 may hold the hydroxyapatite particles and/or other materials in place in socket 21 during healing and prevent migration of cells and/or connective tissue into socket 21. However, connective tissue (e.g., gingival tissue 19) may form a weak attachment with the textured surface of second layer 16, without growing through membrane 10. The attachment may be weak enough that membrane 10 may be removed after healing without significant trauma but may be strong enough to prevent dehiscence.

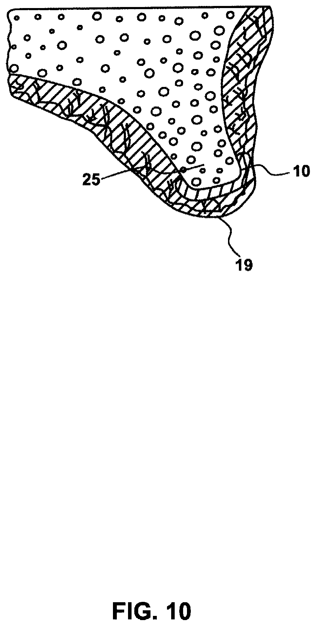

FIG. 10 shows a typical tooth extraction site after healing, but prior to removal of membrane 10. As shown in FIG. 10, the alveolar ridge profile 25 may be preserved and the gingival tissue 19 may be completely healed over ridge 25. Membrane 10 may be removed by making a small incision (not shown) in gingival tissue 19 to expose a portion of layer 23. The layer 23 may then be pulled out with forceps or the like. The material may typically be easily pulled out without trauma to the patient because the attachment of the connective tissue to the textured surface is weak.

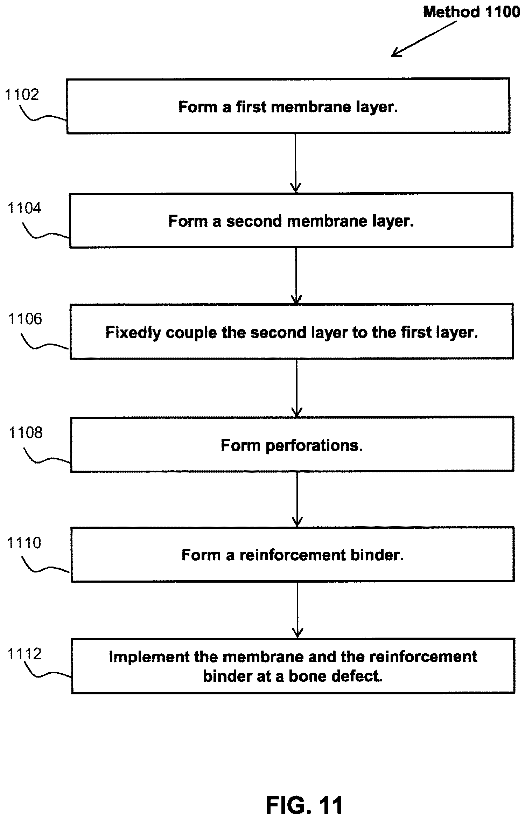

FIG. 11 illustrates a method 1100 for guiding bone and tissue regeneration for a bone defect with a membrane. The operations of method 1100 presented below are intended to be illustrative. In some implementations, method 1100 may be accomplished with one or more additional operations not described, and/or without one or more of the operations discussed. Additionally, the order in which the operations of method 1100 are illustrated in FIG. 11 and described herein is not intended to be limiting.

At an operation 1102, a first layer of the membrane configured to contact bone may be formed. The first layer may include pores configured to promote ingrowth of bone regenerating cells into the first layer. In some implementations, operation 1102 may be performed by a layer the same as or similar to first layer 14 (shown in FIG. 3 and described herein).

At an operation 1104, a second layer of the membrane configured to substantially prevent fibrous connective tissue from growing into the bone defect may be formed.

At an operation 1106 the second layer may be fixedly coupled to the first layer. In some implementations, operation 1106 may be performed by layers the same as or similar to second layer 16 (shown in FIG. 4 and described herein) and first layer 14 (shown in FIG. 3 and described herein).

At an operation 1108, one or more perforations through the membrane may be formed or they can be pre-formed. The perforations comprise co-axial through-holes having common dimensions through the first layer and the second layer. The perforations may be configured to enhance ossification. In some implementations, forming the one or more perforations may include forming the perforations with a substantially circular cross section having a diameter of about 0.1mm or larger. In some implementations, forming the one or more perforations may include forming the perforations with a substantially circular cross section having a diameter of about 0.5mm to about 1.0mm. In some implementations, operation 1108 may comprise determining one or more of a size, a density, a spacing, and/or other characteristics of the perforations based on one or more of a material that forms the membrane, a thickness of the membrane, a size of the membrane, and/or other factors. In some implementations, operation 1108 may comprise forming one or more secondary perforations configured to receive fasteners configured to hold the membrane in place at the bone defect. The fasteners may be pins, for example, and/or other fastening devices. In some implementations, operation 1108 may be performed by perforations the same as or similar to perforations 18 (shown in FIG. 1 and described herein).

At an operation 1110, a reinforcement binder may be formed. The reinforcement binder may comprise multiple elongated members extending from a junction. The elongated members may include a first elongated member, for example, having a free end that extends away from the junction with a predrilled hole formed therein. In some implementations, operation 1110 may be performed by a reinforcement binder the same as or similar to reinforcement binder 12 (shown in FIG. 1 and described herein).

At an operation 1112, the membrane and the reinforcement binder may be implemented at the bone defect. Implementing the membrane and the reinforcement binder at the bone defect may comprise placing the membrane and the reinforcement binder over the bone defect, receiving a fastener with the predrilled hole that passes through at least one of the first or second layer of the membrane, and coupling the membrane and the reinforcement binder with surrounding bone and holding the membrane in place at the bone defect via the fastener. In some implementations the reinforcement binder may be formed between the first layer and the second layer of the membrane. In some implementations, the reinforcement binder may be formed from titanium and may receive a shape imparted to the reinforcement binder via bending by a user. In some implementations, operation 1112 may be performed by a membrane the same as or similar to membrane 10 (shown in FIG. 1 and described herein) and a reinforcement binder the same as or similar to reinforcement binder 12 (shown in FIG. 1 and described herein.

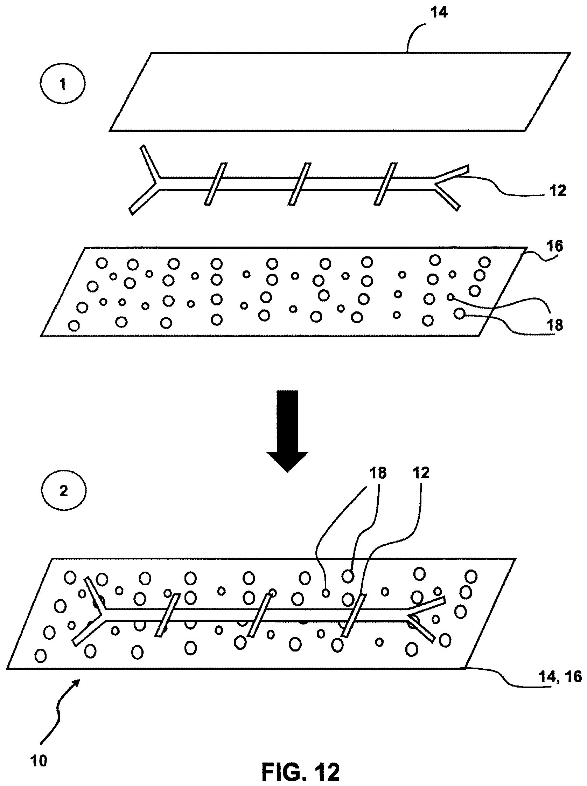

As described above and illustrated in FIG. 12, membrane 10 may be configured such that first layer 14 is a thin layer of expanded PTFE having pores in a size range of about 30 microns to about 1000 microns, but no perforations. In these implementations (not claimed), first layer 14 is not perforated, but rather covers second layer 16 of dense PTFE, which is perforated (e.g., perforations 18). These implementations may create a (e.g., laminated) two-layer membrane, with one layer (e.g., first layer 14) comprising an open-structured mesh and the second layer (e.g., second layer 16) comprising a high density, cell occlusive material. Such implementations may still allow communication of large molecules between the periosteum and the underlying bone graft, just as when perforations 18 are through both layers of membrane 10. As shown in FIG. 12, such implementations, may include a reinforcement binder 12 (e.g., a flexible titanium reinforcement binder) as described herein.

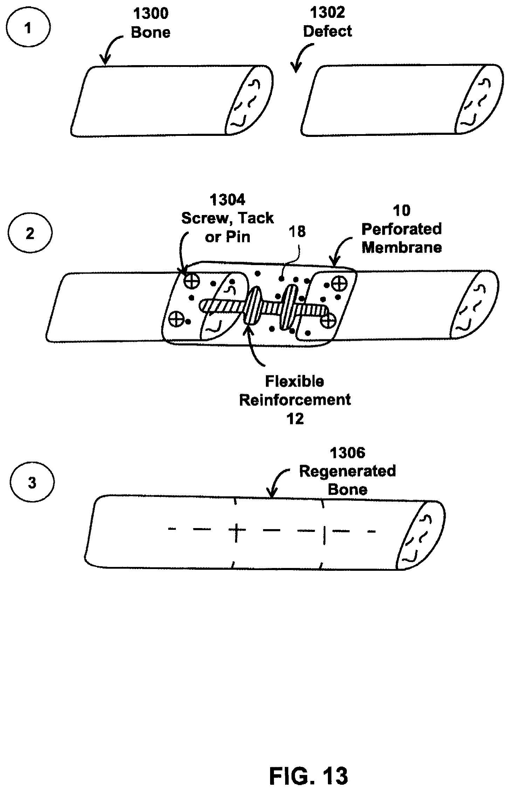

FIG. 13 illustrates treatment of a discontinuity defect with membrane 10. FIG. 13 illustrates a bone 1300 and a defect 1302. Membrane 10 may include perforations 18, reinforcement binder 12, and/or other components. Membrane 10 may be placed across, over, and/or around defect 1302 and fastened in place at defect 1302 by screws, tacks, pins, and/or other fasteners 1304. Membrane 10 may facilitate bone regeneration 1306 as described herein.

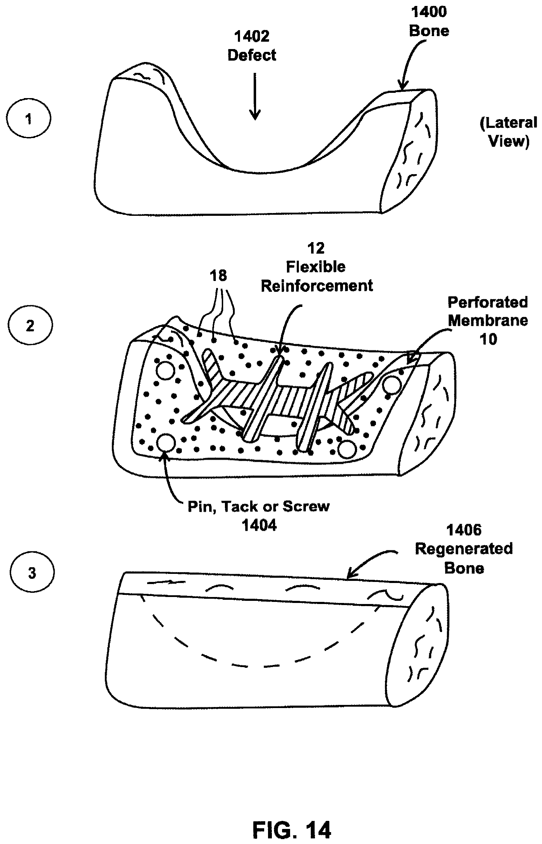

FIG. 14 illustrates treatment of a vertical defect with membrane 10. FIG. 14 illustrates a bone 1400 and a defect 1402. Membrane 10 may include perforations 18, reinforcement binder 12, and/or other components. Membrane 10 may be placed across, over, and/or around defect 1402 and fastened in place at defect 1402 by screws, tacks, pins, and/or other fasteners 1404. Membrane 10 may facilitate bone regeneration 1406 as described herein.

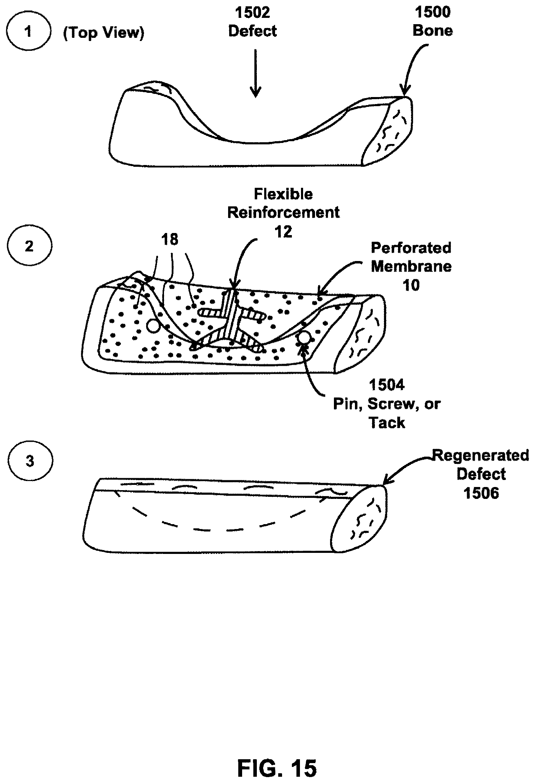

FIG. 15 illustrates treatment of a horizontal defect with membrane 10. FIG. 15 illustrates a bone 1500 and a defect 1502. Membrane 10 may include perforations 18, reinforcement binder 12, and/or other components. Membrane 10 may be placed across, over, and/or around defect 1502 and fastened in place at defect 1502 by screws, tacks, pins, and/or other fasteners 1504. Membrane 10 may facilitate bone regeneration 1506 as described herein.

## Claims

1. A membrane configured to guide bone and tissue regeneration for a bone defect, the membrane (10) comprising: a first layer (14) made of expanded polytetrafluoroethylene (PTFE) configured to contact bone and including pores (15) in a size range of 30 micrometres to 1000 micrometres, and a second layer (16) made of an unsintered, substantially unexpanded polytetrafluoroethylene having a denser structure compared to the first layer (14) and having a limited porosity that allows for the passage of ions and other small molecules necessary for cellular nourishment and waste transport, the pore channels of the second layer (16) having a nominal pore channel size of less than 5 micrometres, and the second layer (16) is fixedly coupled to the first layer (14), comprising secondary perforations (20) to receive fasteners (1204, 1304, 1404 and 1504) configured to hold the membrane (10) in place at the bone defect, **characterized in that** the membrane comprises first perforations (18) extending through the membrane (10) and the membrane (10) being configured to be spread over the bone defect, the first perforations (18) comprising substantially circular co-axial through-holes having common dimensions through the first layer (14) and the second layer (16) which through-holes have diameters being at least 0.1 mm and smaller than 3.0 mm.

2. The membrane as claimed in claim 1, wherein the perforations have a diameter of about 0.5mm to about 1.0mm.

3. The membrane as claimed in claim 1, wherein the membrane (10) has a thickness from about 0.125 mm to about 0.25 mm.

4. The membrane as claimed in any of the claims 1 to 3, **characterized in that** the size of the perforations (18) varies across the membrane (10).

5. The membrane as claimed in claim 1, **characterized in that** the fasteners (1204, 1304, 1404 and 1504) are pins, tacks, sutures or screws.

6. The membrane as claimed in any of the claims 1 to 5, **characterized by** comprising a reinforcement binder (12) configured to be placed over the bone defect and couple with surrounding bone, the reinforcement binder (12) comprising multiple elongated members (30) extending from a junction (32), the elongated members (30) including a first elongated member (31) having a free end (33) that extends away from the junction (32) with a predrilled hole (34) formed therein, the predrilled hole (34) configured to receive a fastener (1204, 1304, 1404 and 1504) that passes through at least one of the first or second layer (14 or 16) of the membrane (10) and holds the membrane (10) in place at the bone defect.

7. The membrane as claimed in claim 6, wherein the reinforcement binder (12) is formed between the first layer (14) and the second layer (16) of the membrane (10).

## Patentansprüche

1. Membran, die konfiguriert ist, um Knochen- und Geweberegeneration für einen Knochendefekt zu führen, wobei die Membran (10) umfasst: eine erste Schicht (14) aus expandiertem Polytetrafluorethylen (PTFE), die für den Kontakt mit Knochen konfiguriert ist und Poren (15) in einem Größenbereich von 30 Mikrometer bis 1000 Mikrometer enthält, und eine zweite Schicht (16) aus einem ungesinterten, im Wesentlichen nicht expandierten Polytetrafluorethylen mit einer dichteren Struktur im Vergleich zu der ersten Schicht (14) und mit einer begrenzten Porosität, die den Durchgang von Ionen und anderen kleinen Molekülen, die für die Zellernährung und den Abfalltransport erforderlich sind, ermöglicht, wobei die Porenkanäle der zweiten Schicht (16) eine nominelle Porenkanalgröße von weniger als 5 Mikrometer aufweisen und die zweite Schicht (16) fest mit der ersten Schicht (14) gekoppelt ist und sekundäre Perforationen (20) umfasst, um Befestigungselemente (1204, 1304, 1404 und 1504) aufzunehmen, die so konfiguriert sind, dass sie die Membran (10) an Ort und Stelle am Knochendefekt halten,
**dadurch gekennzeichnet, dass** die Membran erste Perforationen (18) umfasst, die sich durch die Membran (10) erstrecken und die Membran (10) so konfiguriert ist, dass sie über den Knochendefekt verteilt wird, wobei die ersten Perforationen (18) im Wesentlichen kreisförmige koaxiale Durchgangslöcher mit gemeinsamen Abmessungen durch die erste Schicht (14) und die zweite Schicht (16) umfassen, wobei die Durchgangslöcher Durchmesser von mindestens 0,1 mm und kleiner als 3,0 mm aufweisen.

2. Membran nach Anspruch 1, wobei die Perforationen einen Durchmesser von etwa 0,5 mm bis etwa 1,0 mm aufweisen.

3. Membran nach Anspruch 1, wobei die Membran (10) eine Dicke von etwa 0,125 mm bis etwa 0,25 mm aufweist.

4. Membran nach einem der Ansprüche 1 bis 3, **dadurch gekennzeichnet, dass** die Größe der Perforationen (18) über die Membran (10) hinweg variiert.

5. Membran nach Anspruch 1, **dadurch gekennzeichnet, dass** die Befestigungsmittel (1204, 1304, 1404 und 1504) Nadeln, Stifte, Nähte oder Schrauben sind.

6. Membran nach einem der Ansprüche 1 bis 5, **dadurch gekennzeichnet, dass** sie einen Verstärkungsbinder (12) umfasst, der konfiguriert ist, um über dem Knochendefekt angeordnet zu werden und mit dem umgebenden Knochen zu koppeln, wobei der Verstärkungsbinder (12) mehrere längliche Elemente (30) umfasst, die sich von einer Verbindungsstelle (32) erstrecken, wobei die länglichen Elemente (30) ein erstes längliches Element (31) aufweisen, das ein freies Ende (33) aufweist, das sich von der Verbindungsstelle (32) weg erstreckt, wobei ein vorgebohrtes Loch (34) darin ausgebildet ist, wobei das vorgebohrte Loch (34) so konfiguriert ist, dass es ein Befestigungselement (1204, 1304, 1404 und 1504) aufnimmt, das durch mindestens eine der ersten oder zweiten Schicht (14 oder 16) der Membran (10) hindurchgeht und die Membran (10) an Ort und Stelle am Knochendefekt hält.

7. Membran nach Anspruch 6, wobei das Verstärkungsbindemittel (12) zwischen der ersten Schicht (14) und der zweiten Schicht (16) der Membran (10) gebildet ist.

## Revendications

1. Membrane configurée pour guider la régénération osseuse et tissulaire pour un défaut osseux, la membrane (10) comprenant : une première couche (14) faite de polytétrafluoroéthylène expansé (PTFE) configurée pour entrer en contact avec l'os et comprenant des pores (15) dans une gamme de taille de 30 micromètres à 1000 micromètres, et une seconde couche (16) faite d'un polytétrafluoroéthylène non fritté, sensiblement non expansé, ayant une structure plus dense que la première couche (14) et ayant une porosité limitée qui permet le passage d'ions et d'autres petites molécules nécessaires à l'alimentation cellulaire et au transport des déchets, les canaux de pores de la seconde couche (16) ayant une taille nominale de canal de pores inférieure à 5 micromètres, et la seconde couche (16) est couplée de manière fixe à la première couche (14), comprenant des perforations secondaires (20) pour recevoir des éléments de fixation (1204, 1304, 1404 et 1504) configurés pour maintenir la membrane (10) en place au niveau du défaut osseux, **caractérisé en ce que** la membrane comprend des premières perforations (18) s'étendant à travers la membrane (10) et la membrane (10) étant configurée pour être étalée sur le défaut osseux, les premières perforations (18) comprenant des trous traversants coaxiaux sensiblement circulaires ayant des dimensions communes à travers la première couche (14) et la seconde couche (16), lesquels trous traversants ont des diamètres d'au moins 0,1 mm et inférieurs à 3,0 mm.

2. Membrane selon la revendication 1, où les perforations ont un diamètre d'environ 0,5 mm à environ 1,0 mm.

3. Membrane selon la revendication 1, où la membrane (10) a une épaisseur d'environ 0,125 mm à environ 0,25 mm.

4. Membrane selon l'une quelconque des revendications 1 à 3, **caractérisée en ce que** la taille des perforations (18) varie sur la membrane (10).

5. Membrane selon la revendication 1, **caractérisée en ce que** les éléments de fixation (1204, 1304, 1404 et 1504) sont des broches, des punaises, des sutures ou des vis.

6. Membrane selon l'une quelconque des revendications 1 à 5, **caractérisée en ce qu'**elle comprend un liant de renforcement (12) configuré pour être placé sur le défaut osseux et se coupler à l'os environnant, le liant de renforcement (12) comprenant de multiples éléments allongés (30) s'étendant depuis une jonction (32), les éléments allongés (30) comprenant un premier élément allongé (31) ayant une extrémité libre (33) qui s'étend à l'écart de la jonction (32) avec un trou pré-percé (34) formé dans celle-ci, le trou pré-percé (34) étant configuré pour recevoir un élément de fixation (1204, 1304, 1404 et 1504) qui passe à travers au moins une de la première ou de la seconde couche (14 ou 16) de la membrane (10) et maintient la membrane (10) en place au niveau du défaut osseux.

7. Membrane selon la revendication 6, où le liant de renforcement (12) est formé entre la première couche (14) et la seconde couche (16) de la membrane (10).
